(19) Europäisches Patentamt
European Patent Office
Office européen des brevets

(11) EP 1 671 643 A1

(12) # EUROPEAN PATENT APPLICATION
published in accordance with Art. 158(3) EPC

(43) Date of publication:
**21.06.2006 Bulletin 2006/25**

(21) Application number: **04772965.2**

(22) Date of filing: **07.09.2004**

(51) Int Cl.:
***A61K 38/20*** *(1995.01)* ***A61K 38/21*** *(1995.01)*
***A61K 38/17*** *(1995.01)* ***A61K 31/7004*** *(2000.01)*
***A61K 31/7088*** *(2000.01)* ***A61P 1/16*** *(2000.01)*
***A61P 31/14*** *(2000.01)*

(86) International application number:
**PCT/JP2004/013283**

(87) International publication number:
**WO 2005/023289 (17.03.2005 Gazette 2005/11)**

(84) Designated Contracting States:
**AT BE BG CH CY CZ DE DK EE ES FI FR GB GR HU IE IT LI LU MC NL PL PT RO SE SI SK TR**

(30) Priority: **08.09.2003 JP 2003315498**

(71) Applicants:
- **Intellectual Property Consulting Inc.
Tokyo 100-0011 (JP)**
- **Hayashi, Norio
Kawanishi-shi,
Hyogo 666-0116 (JP)**

(72) Inventor: **HAYASHI, Norio
Kawanishi-shi, Hyogo 6660116 (JP)**

(74) Representative: **Helbing, Jörg et al
Patentanwälte
von Kreisler-Selting-Werner,
Postfach 10 22 41
50462 Köln (DE)**

(54) **MEDICINAL COMPOSITION FOR TREATING CHRONIC HEPATITIS C**

(57) The present invention provides a pharmaceutical composition for treating chronic hepatitis C **characterized by** comprising at least one active ingredient selected from the group consisting of IL-15, myeloid dendritic cell maturation stimulators (for example, CpG oligo deoxynucleotide, GM-CSF, IL-4, LPS, CD40L, polyI:C, TNF-α and IFN-γ) and lectin-binding substances (for example, mannose carbohydrate, fucose carbohydrate and anti-lectin antibody); and a method of treating chronic hepatitis C by using such active ingredient(s). In the above pharmaceutical composition and therapeutic method, it is possible to use INF-α together with these active ingredients.

EP 1 671 643 A1

## Description

### Technical Field

The present invention relates to a method and a pharmaceutical composition for treating chronic hepatitis C.

### Background Art

A hepatitis C virus (HCV) is a single stranded plus sense RNA virus belonging to the Flavivirus family, and causes persistent infection in 70% or more of infected patients. A most important feature of HCV persistent infection is a possibility of progress of hepatic diseases from mild hepatitis to hepatic cirrhosis and hepatic cell carcinoma. The number of patients suffering from HCV positive hepatic cell carcinoma is increasing on a global basis, and chronic HCV infection is becoming a serious problem.

For preventing progress of hepatic diseases and subsequent onset of hepatic cell carcinoma, it is necessary to eradicate HCV from a HCV-infected patient. For this purpose, a therapy combining IFN-$\alpha$ and ribavirin is currently used as a standard therapeutic method for chronic HCV infection (Japanese Patent Application Laid-Open (JP-A) No. 11-152231). This therapeutic method remarkably improved the probability of HCV eradication as compared with a therapy using singly IFN-$\alpha$, however, half or more of patients are not cured by this combination therapy.

One mechanism of HCV persistent infection is an ability of HCV of escaping from an immune response of a host (Proc. Natl. Acad. Sci. USA 92(7): 2755-9, 1995; Science 258 (5079): 135-40). Studies to date have clarified that a functional disorder of immune response cells is observed in a chronic HCV-infected patient (J. Immunol., 169(6): 3447-3458, 2002; Clin. Exp. Immunol., 109(3): 451-457, 1997), and it is believed that HCV suppresses an immune response by various method. It is suggested that HCV has a strategy of escaping from an immune surveillance system, by, for example, allowing an antigen presenting function not to act (J. Immunol. 162: 5584-5591, 1999) or disturbing a CD4 or CD8 T cell response (J. Immunol. 169: 3447-3458, 2002; Hepatology 33: 267-276, 2001).

One important feature of HCV persistent infection is that HCV suppresses a dendritic cell (DC) function. A dendritic cell is a most strong antigen presenting cell, and bears various immune responses (Banchereau, et al., Nature 392(6673): 245-252, 1998: Blood, 90(9): 3245-3287, 1997). Blood dendritic cells are constituted mainly two subsets of myeloid dendritic cells and plasmacyte dendritic cells. A myeloid dendritic cell (MDC) is characterized by having a strong immune stimulating property on both primary and secondary T cell responses on a virus, and when stimulated, MDC releases IL-12 or TNF-$\alpha$ and preferentially induces a Th1 response. A plasmacyte (lymphocyte) dendritic cell (PDC), when infected with a virus, releases a large amount of I type IFN and induces mainly Th2 polarization.

It is shown that, in chronic HCV infection, a T cell stimulating ability of a dendritic cell is defective (J. Immunol. 162: 5584-5591, 1999; Gastroenterology 120: 512-524, 2001), and it is suggested from this fact that a dendritic cell is correlated with immune function disorder induced by HCV. Several studies using reverse transcription (RT)-PCR teach the presence of a HCV genome in blood cells including dendritic cells. However, this method cannot clarify whether HCV invades a cell or only adheres to its surface. It is suggested that direct infection of HCV on a blood dendritic cell has some engagement with dysfunction of a dendritic cell, however, it is up to now unclear which dendritic cell subset is sensitive to HCV

A dendritic cell can, when stimulated with IFN$\alpha$, express MICA/B to activate an NK cell. An MHC class I related A chain and B chain (MICAB) are ligands of NKG2D transmitting positive intracellular signals in an NK cell. An NK cell is a main component of inherited immunity constituting the front of defense against various causative factors by directly killing infected cells. Activation of NK cells also exerts an influence on the subsequent adaptive immune response, by releasing various cytokines, for example, IFN$\gamma$. Though MICA/B, unlike traditional class I MHC, is not expressed in most normal cells, up-regulated in a lot of epithelium tumor cells, cells infected with human Cytomegalovirus, and "stressed" cells. Therefore, it is believed that MICAB plays an important role in excluding transformed cells and infected cells, by activating NK cells.

It is reported that patients infected with hepatitis C virus are deficient significantly in induction of MICAB by stimulation with IFN$\alpha$ (J. Immunol. 170: 1249-1256, 2003). Namely, it is believed that, in chronic HCV-infected patients, a defect of MICA/B expression in dendritic cells exerts an influence on activity of NK cells and the subsequent T cells, however, its mechanism is not clear up to now.

Prior technology literature information related to the present invention is described below.

Patent document 1: Japanese Patent Application Laid-Open (JP-A) No. 11-152231
Non-patent document 1: J. Immunol. 162: 5584-5591, 1999
Non-patent document 2: Gastroenterology 120: 512-524, 2001
Non-patent document 3: J. Immunol. 170: 1249-1256, 2003

## Disclosure Of The Invention

An object of the present invention is to provide a method and a pharmaceutical composition, for augmenting a function of an immune system, treating chronic hepatitis C and preventing sideration of hepatic cirrhosis and hepatic cell carcinoma, in chronic HCV-infected patients, by clarifying a mechanism of HCV persistent infection, particularly, a mechanism of MICA/B expression induced by IFN$\alpha$ in dendritic cells.

The present inventors have found that when a dendritic cell of a chronic HCV-infected patient is stimulated with IL-15, expression of MICAB is induced and NK cells are activated. Also, the present inventors have clarified that a pseudo type vesicular stomatitis virus (VSV) infects immature MDC but does not infect MDC matured by imparting a maturation stimulator, further that a lectin-containing molecule on MDC plays an important role for pseudo type VSV invasion. The present invention has been completed based on such knowledge.

That is, the present invention provides a pharmaceutical composition for treating chronic hepatitis C, comprising at least one active ingredient selected from the group consisting of IL-15, myeloid dendritic cell maturation stimulators and lectin-binding substances. Preferably, such a pharmaceutical composition is used together with IFN-$\alpha$ in treatment of chronic hepatitis C.

According to a preferable embodiment of the present invention, there is provided a pharmaceutical composition for treating chronic hepatitis C, comprising IL-15. Preferably, the composition of the present invention comprising IL-15 is used together with IFN-$\alpha$ in treatment of chronic hepatitis C.

Treatment of chronic hepatitis C means decreasing persistently infected hepatitis C virus, preferably, eradication thereof, and by this, sideration of hepatic cirrhosis and hepatic carcinoma can be preventing in chronic HCV-infected patients.

From another standpoint, the present invention provides a pharmaceutical composition for treating chronic hepatitis C, comprising a myeloid dendritic cell maturation stimulator. Preferably, the composition comprising a myeloid dendritic cell maturation stimulator is used together with IFN-$\alpha$ in treatment of chronic hepatitis C. Preferably, the myeloid dendritic cell maturation stimulator is selected from the group consisting of CpG oligo deoxynucleotide, GM-CSF, IL-4, LPS, CD40L, polyI:C, TNF-$\alpha$ and IFN-$\gamma$.

From another standpoint, the present invention provides a pharmaceutical composition for treating chronic hepatitis C, comprising a lectin binding substance. Preferably, the composition comprising a lectin binding substance is used together with IFN-$\alpha$ in treatment of chronic hepatitis C. Preferably, the lectin-binding substance is selected from the group consisting of mannose carbohydrates, fucose carbohydrates and anti-lectin antibodies.

The present invention provides also a pharmaceutical composition for preventing hepatic cirrhosis and hepatic cell carcinoma, comprising at least one active ingredient selected from the group consisting of IL-15, myeloid dendritic cell maturation stimulators and lectin-binding substances.

The present invention provides also a method of treating chronic hepatitis C, comprising administration to a patient of an effective amount of at least one active ingredient selected from the group consisting of IL-15, myeloid dendritic cell maturation stimulators and lectin-binding substances.

Further, the present invention provides also use of IL-15, myeloid dendritic cell maturation stimulator and/or lectin-binding substance in manufacture of a medicine for treatment of chronic hepatitis C.

## Brief Description Of Drawings

Fig. 1 shows induction of expression of MICA/B by various cytokines in dendritic cells.
Fig. 2 shows induction of expression of MICA/B by IL-15 and IFN$\alpha$.
Fig. 3 shows activation of NK cells by a dendritic cell stimulated with IL-15 or IFN$\alpha$.
Fig. 4 shows activation of NK cells by a dendritic cell stimulated with IL-15 or IFN$\alpha$.
Fig. 5 shows a result of an experiment investigating NK cell activation by IL-15.
Fig. 6 shows a result of an experiment in which NK cells and dendritic cells stimulated with IL-15 are co-cultured in the trans well system.
Fig. 7 shows inhibition of NK cell activation by an anti-MICA/B antibody.
Fig. 8 shows inhibition of NK cell activation by an anti-MICA/B antibody.
Fig. 9 shows production of IL-15 by dendritic cells stimulated with IFN$\alpha$.
Fig. 10 shows disturbance of MICA/B expression by an anti-IL-15 antibody or an anti-IL-15R$\alpha$ antibody.
Fig. 11 shows production of IFN$\alpha/\beta$ by dendritic cells stimulated with IL-15.
Fig. 12 shows disturbance of expression of IL-15 inductive MICA/B by an anti-IFN$\alpha/\beta$R antibody.
Fig. 13 shows the number of dendritic cells in blood of an HCV-infected patient and a control.
Fig. 14 shows a result of inoculation of pseudo type VSV-E1E2 on MDC and PDC.

Fig. 15 shows a result of inoculation of pseudo type VSV-E1E2 on Mo-DC.
Fig. 16 shows the copy number of HCV RNA in MDC, Mo-DC and PDC.
Fig. 17 shows an influence of various MDC maturation stimulators exerted on infection of pseudo type VSV on MDC.
Fig. 18 shows an influence of mannan exerted on infection of pseudo type VSV on MDC.
Fig. 19 shows an influence of mannan exerted on infection of pseudo type VSV on MDC.
Fig. 20 shows an influence of mannan exerted on infection of pseudo type VSV on HepG2 cells.

Best Modes For Carrying Out The Invention

Since it is known that there is a deficiency in MICA/B expression in a dendritic cell in a chronic HCV-infected patient, the present inventors stimulated dendritic cells from healthy donors and chronic HCV-infected patients with various cytokines and checked expression of MICA/B. As shown in Example 1, in dendritic cells of healthy donors, expression of MICA/B is induced by IFNα, IFNβ or IL-15, while in dendritic cells of HCV-infected patients, expression of MICA/B is induced by IL-15 but not induced by IFNα or IFNβ. Further, when dendritic cell of HCV-infected patients are stimulated with IL-15, cytophathy activity of NK cells and IFNγ production are augmented. Furthermore, as shown in Example 4, it has been clarified that when dendritic cells of healthy donors are stimulated with IFNα, production of IL-15 is induced, while not induced in the case of dendritic cells of HCV-infected patients. These results have shown that dendritic cells from HCV-infected patients cannot induce expression of MICA/B and accordingly cannot activate NK cells since an ability of producing IL-15 by stimulation with IFNα is disturbed in the dendritic cells of HCV-infected patients. This is believed to be one reason for the fact that eradication of HCV by IFNα therapy is difficult in chronic HCV-infected patients.

Namely, the present invention provides a pharmaceutical composition for treating chronic hepatitis C, characterized by comprising IL-15.

IL-15 has initially been isolated as a factor of promoting proliferation of a T cell strain, and a sequence of cDNA of human IL-15 has been reported (Science 264: 965-968, 1994). IL-15 is one of cytokines correlated with an immune response, and is a T cell growth factor which can stimulate proliferation and differentiation of B cells, T cells, natural killer (NK) cells and lymphocyte activation killer (LAK) cells, to induce CTL activity. mRNA of IL-15 is expressed in a lot of tissues, and significantly expressed particularly in monocytes and macrophages. In vivo, IL-15 causes multilateral actions on various immune cells, and plays a role in augmentation of an immune response of an organism.

Recent studies have shown that IL-15 positively controls a function and maturation process of dendritic cells, to generate an effective pathogen-specific or tumor-specific CTL response (J. Immunol., 169: 4928, 2002; J. Exp. Med., 194: 1013, 2001; Nature Immunol., 12: 1138, 2001). Further, a dendritic cell produces IL-15 in response to inflammatory stimulation, and by this manifests an immune control function (J. Immunol., 167: 1179, 2001; J. Immunol., 169: 4279, 2002). However, it has been utterly unclear whether IL-15 exerts some kind of influence on an ability of a dendritic cell of regulating an NK cell function or not. In the present invention, it has been shown that a dendritic cell, when stimulated with IL-15, expresses MICA/B and activates a static NK cell and that this action is completely dependent on an MICA/B-NKG2D mutual action. Though it is known that IL-15 acts as an NK cell stimulator, it has been clarified, in the present invention, that IL-15 itself has only a secondary influence on an NK cell function.

IL-15 used in the present invention can be obtained by various methods. For example, primary culturing cells or cell strain producing IL-15 is cultured and IL-15 can be isolated and purified from the cultured substance, alternatively, IL-15 can be produced recombinantly by a method known in the art based on a known gene sequence (for example, sequence described in the above-mentioned Science 264: 965-968, 1994). In thus obtained IL-15, parts of its amino acid sequence (for example, 1 to 30, 1 to 20, 1 to 10, 1 to several (e.g., 5), 1 to 2 amino acid residues) may be deleted or substituted, and other amino acid sequences may be partially (for example, 1 to 30, 1 to 20, 1 to 10, 1 to several (e.g., 5), 1 to 2 amino acid residues) inserted, providing IL-15 has an ability of stimulating dendritic cells. Also, it may have a saccharide chain different from natural saccharide chains. DNA having a sequence having deletion or substitution of a sequence coding IL-15 or parts thereof (for example, 1 to 30, 1 to 20, 1 to 10, 1 to several (e.g., 5), 1 to 2 bases) can be incorporated into a suitable expression vector and this can be introduced into an eukaryote or prokaryote cell, to express an IL-15 protein. Examples of host cells which can be used for expressing a recombinant protein include, but not limited to, prokaryote hosts such as E. coli, Bacillus subtilis and the like, and eukaryote hosts such as yeast, fungus, insect cell, mammal cell and the like. Preferably, mammal cells are used.

A vector comprises a promoter region driving gene expression, further, may comprise transcription and translation control sequences, for example, TATA box, capping sequence, CAAT sequence, 3' non-coding region, enhancer and the like. Examples of the promoter, when used in a prokaryote host, include a bla promoter, cat promoter and lacZ promoter, and when used in an eukaryote host, include a TK promoter of herpes virus, SV40 initial promoter, yeast glycolysis system enzyme gene sequence promoter and the like. Examples of the vector include, but not limited to, pBR322, pUC118, pUC119, λgt10, λgt11, pMAM-neo, pKRC, BPV, vaccinia, SV40, 2-micron and the like. Further, a vector can be formulated so that secretion of a recombinant protein is expressed using a signal sequence or so that a recombinant protein is expressed in the form of fused protein with other protein. Such formulation of an expression vector is well known in the art.

A vector so formulated as to express IL-15 can be introduced into a suitable host cell by transformation, transfection, conjugation, protoplast fusion, electroporation, particle gun technology, calcium phosphate precipitation, direct micro injection and the like. An IL-15 protein can be obtained by growing cells containing a vector in a suitable medium to produce recombinant proteins, recovering the desired recombinant protein from the cells or medium, and purifying this. Purification can be conducted by using size exclusion chromatography, HPLC, ion exchange chromatography, immune affinity chromatography and the like.

From another standpoint of the present invention, it has been found that the number of MDC and PDC decreases and its function has a defect in chronic HCV-infected patients. As shown in Example 7 described later, the absolute number of sum of DC and PDC, and precursor cells of them is lower than that of control donors in HCV-infected patients, and particularly lower in patients suffering from hepatitis. Though how dendritic cells are in vivo generated from precursor cells has not been clarified yet, it is believed, from these results, that small number of dendritic cells in blood of a chronic hepatitis C patient is ascribable at least partially to reduction of precursor cells or precursors of dendritic cells.

From still another standpoint of the present invention, the present inventors have inoculated a pseudo type vesicular stomatitis virus (VSV) coated with chimera HCV envelope glycoprotein on a dendritic cell, and investigated whether the virus invaded into the cell or not. The pseudo type VSV can discriminate an infected cell by fluorescence since the VSV has a green fluorescent protein (GFP) reporter gene in its genome. As shown in Example 9, it has been clarified that a pseudo type VSV invades MDC derived from a HCV negative donor and monocyte-derived dendritic cells (MoDC) but does not invade PDC. Further, as shown in Example 10, it has been clarified that a pseudo type VSV infects immature MDC but does not infect MDC matured by imparting a maturation stimulator.

Namely, in the present invention, it has been found that chronic hepatitis C can be treated by imparting a myeloid dendritic cell maturation stimulator. Preferably, the myeloid dendritic cell maturation stimulator is used together with IFN-α in the treatment of chronic hepatitis C. "Myeloid dendritic cell maturation stimulator" is not particularly restricted providing it can stimulate maturation of myeloid dendritic cells, and examples thereof include CpG oligo deoxynucleotide, GM-CSF, IL-4, LPS, CD40L, polyI:C, TNF-α and IFN-γ. The CpG oligo deoxynucleotide is an oligo nucleotide comprising a non-methylated CpG motif, and is known to have an ability of stimulating an immune response, particularly a Th1 response, to induce activation of B cells, NK cells and antigen presenting cells. In the present invention, it has been clarified that CpG oligo deoxynucleotide stimulates MDC to mature MDC, lowering sensitivity of MDC against pseudo type VSV. All of GM-CSF, IL-4, TNF-α and IFN-γ are generally used as a factor capable of deriving a dendritic cell from a precursor cell. LPS (lipopolysaccharide, a component of gram negative bacterium cell wall), CD40L and polyI:C are well-known as an activator for a dendritic cell. Any of these factors can be used instead of CpG oligo deoxynucleotide. Two or more myeloid dendritic cell maturation stimulators may be combined. Further, the above-mentioned myeloid dendritic cell maturation stimulator can be used in combination with IFN-α or ribavirin in treatment of chronic hepatitis C.

The present inventors have further clarified that a lectin-comprising molecule on MDC plays an important role on invasion of pseudo type VSV. As shown in Example 12, invasion of pseudo type VSV-E1E2 into MDC is inhibited by mannan. Namely, it has been found that chronic hepatitis C can be treated by inhibiting a mutual action between HCV and a lectin-comprising molecule on MDC using a lectin-binding substance. "Lectin-binding substance" is not particularly restricted providing it is a substance binding to a lectin-comprising molecule on MDC to inhibit a mutual action between HCV and the lectin-comprising molecule on MDC, and for example, mannose carbohydrates, fucose carbohydrates, anti-lectin antibodies and the like can be used. Two or more lectin-binding substances may be used in combination. Further, the above-mentioned lectin-binding substance can be used in combination with IFN-α or ribavirin in treatment of chronic hepatitis C.

For manufacture of a pharmaceutical composition of the present invention, IL-15, myeloid dendritic cell maturation stimulator and/or lectin-binding substance, if necessary, further with IFN-α added, can be formulated together with a pharmaceutically acceptable carrier well-known in the art, by mixing, dissolution, granulation, tabletting, emulsification, encapsulation, freeze drying and the like. When prescribed as a pharmaceutical composition, for example, active ingredients are compounded in an amount of 0.1 to 99.9 wt% in its composition. When the active ingredient used in the present invention form a pharmaceutically acceptable salt, use in such salt form is also included in the range of the present invention.

For oral administration, IL-15, myeloid dendritic cell maturation stimulator or lectin-binding substance can be formulated into a dosage form such as tablet, pill, sugar-coated tablet, soft capsule, hard capsule, solution, suspension, emulsion, gel, syrup, slurry and the like, together with a pharmaceutically acceptable solvent, excipient, binder, stabilizer, dispersing agent.

For parenteral administration, IL-15, myeloid dendritic cell maturation stimulator or lectin-binding substance can be formulated into a dosage form such as injection solution, suspension, emulsion, cream, ointment, inhalant, suppository and the like, together with a pharmaceutically acceptable solvent, excipient, binder, stabilizer, dispersing agent. In recipe for injection, a pharmaceutical composition of the present invention can be dissolved in an aqueous solution, preferably, a physiologically compatible buffering solution such as Hank's solution, Ringer solution, physiological salt buffering solution and the like. Further, the composition can take a form such as suspension, solution, emulsion or the like, in an oily or aqueous vehicle. Alternatively, it may also be permissible that a pharmaceutical composition is produced in the form of powder and an aqueous solution or suspension is prepared using sterilized water and the like before use. For administration by inhalation, a therapeutic agent of the present invention can be pulverized to give a powder mixture together with a suitable base agent such as lactose, starch and the like. A suppository can be produced by mixing a therapeutic agent of the present invention with a conventional suppository base agent such as cacao butter and the like. Further, a pharmaceutical composition of the present invention can be enclosed in a polymer matrix and the like, to prescribe a preparation for sustained release.

The dosage and dosage regimen for administering a pharmaceutical composition of the present invention can take various embodiments depending on the age of a patient, the condition and extent of a disease, and can be determined by those skilled in the art. In particular, the preferable administration method can take various embodiments depending on specific medicinal recipes of IL-15, myeloid dendritic cell maturation stimulator or lectin-binding substance, and an immune condition of a patient to be treated.

Further, the pharmaceutical composition of the present invention is particularly useful for use in administration in combination with INF-α. In a treatment combined with INF-α, INF-α and IL-15, myeloid dendritic cell maturation stimulator or lectin-binding substance are administered simultaneously or sequentially so that a synergistic effect can be performed in treatment of chronic hepatitis C. An interval of from several days to several months may also be present between administrations. Administration may be oral, parenteral or a combination of them. Parenteral means, for example, intravenous, subcutaneous, intradermal or intramuscular administration. The composition of the present invention may also be administered in the form of depot recipe.

Though the dosage and dosage frequency vary depending on the dosage form and administration route, and the condition, age and body weight of a patient, in general, the pharmaceutical composition of the present invention can be administered once to several times a day so that the amount of each active ingredient is in the range of from about 0.001 mg to 1000 mg, preferably in the range from about 0.01 mg to 10 mg, based on 1 kg of body weight per day.

The composition of the present invention may also be provided in the form of kit comprising INF-α, and IL-15, myeloid dendritic cell maturation stimulator or lectin-binding substance which is useful for treatment in combination with INF-α, or in the form of once-administration package. The kit or package may comprise a guide for use of a pharmaceutical composition according to the present invention.

## EXAMPLES

The present invention will be illustrated more in detail by the following examples, but the scope of the invention is not limited to these examples.

In Examples 1 to 6, the following materials and methods were used.

(A) Preparation of monocyte-derived DC from PBMC

15 healthy volunteers and 20 chronic HCV-infected patients were used as test subjects. All HCV-infected patients were positive for both a serum anti-HCV antibody and HCV-RNA, and did not show a proof for infection of other type virus or hepatic disease. Serum alanine amino transferase (ALT)level was periodically measured, and HCV-infected patients were classified into two groups depending on the extent of necrotic inflammatory hepatic disease. Patients (n=10) showing persistent or fluctuant ALT increase were defined to be included in CH-1 group, and other patients (n=10) persistently showing normal ALT for 2 years or more were defined to be included in CH-2 group. Monocyte-derived DCs were produced from peripheral vein blood of the healthy volunteers and chronic HCV-infected patients. Simply, PBMC isolated by Ficoll Hypaque density centrifugation was centrifugally separated by three layer density gradient (1.076, 1.059 and 1.045 g/mL) of percoll (Sigma Aldrich, St Louis, MO). An interlayer fraction comprising highly purified monocytes was inoculated on a 24-well culture dish at a density of $5.0 \times 10^5$/well. After incubation for 45 minutes, non-adhesive cells were removed, and adhesive cells were cultured in an I scope-modified Eagle medium (Gibco-BRL Life Technologies, Inc., Gaithersberg, MD) comprising 10% PCS, 10 U/mL penicillin/streptomycin and 2 mmol/L L-glutamine and supplemented with GM-CSF (1000 U/mL, Kirin Brewery Co., Ltd.) and IL-4 (500 U/mL: Strathmaim Biotech., Hannover, Germany). For stimulation of DC, the following reagents were used: IFNα (Sumitomo Seiyaku K.K.), IFNβ (Toray Industries, Inc.), IL-15, IL-12, IL-18 and TNFα (R&D Systems, Mineapolis, MN).

(B) Flow cytometory analysis of MICAB expression in DC

DCs ($5 \times 10^5$) were incubated at 4°C for 30 minutes together with an anti-MICAB monoclonal antibody (6D4) (Science 279: 1737, 1998). Next, the cells were washed, and incubated at 4°C for 30 minutes using FITC-labeled mouse anti-goat IgG (BD-Pharmingen, San Diego, CA) as a secondary antibody. Next, the cells were washed twice, and immobilized with a 2% p-formaldehyde solution. The cells were analyzed by flow cytometory using FACScan system (BD-Pharmingen), and the data were analyzed using CELL Quest (trade name) software.

(C) Analysis of mRNA expression in DC by RT-PCR

Total RNAs (1 μg) were extracted using ISOGEN (Nippon Gene CO., LTD.), and 80 pmol of random primer (Takara Shuzo Co., Ltd.) and 10 mmol/L each deoxynucleotide triphosphate were added, and the mixture was incubated at 95°C for 5 minutes and quenched on ice. The mixture was mixed with 50 mmol/L Tris-HCl, 75 mmol/L KCl, 10 mmol/L DTT, 3 mmol/L $MgCl_2$ and 100 U Moloney mouse leukemia virus reverse transcriptase (Gibco-BRL), and the mixture was incubated at 37°C for 50 minutes.

The reaction was terminated by heating at 70°C for 15 minutes. The resultant cDNA was used in a reaction mixture comprising 10 pmol of each sense and anti-sense primer, 10 mmol/L Tris-HCl, 50 mmol/L KCl, 1.5 mmol/L $MgCl_2$, each 2.5 mmol/L deoxynucleotide triphosphate and 2.5 U TaqDNApolymerase (Takara Shuzo Co., Ltd.).

The following primers were used: MIC sense: 5'-ACACCCAGCAGTGGGGGAT-3' (SEQ ID No: 1); MICA anti-sense: 5'-GCAGGGAATTGAATCCCAGCT-3' (SEQ ID No: 2); MICB anti-sense: 5'-AGCAGTCGTGAGTTTGCCCAC-3' (SEQ ID No: 3); IL-15 sense: 5'-TAAAACAGAAGCCAACTG-3' (SEQ ID No: 4); IL-15 anti-sense: 5'-CAAGAAGTGTTGATGAACAT-3' (SEQ ID No: 5); IL-15Rα sense: 5'-GTCAAGAGCTACAGCTTGTAC-3' (SEQ ID No: 6); IL-15Rα anti-sense: 5'-GGTGAGCTTTCTCCTGGAG-3' (SEQ ID No: 7)

A proliferation protocol is as described below: modification (MIC: 35 cycles, 95°C, 60 seconds, IL-15: 30 cycles, 99°C, 60 seconds, IL-15Rα: 35 cycles, 99°C, 60 seconds), next, annealing process (MIC: 56°C, 60 seconds, IL-15: 60°C, 30 seconds, IL-15Rα: 55°C, 30 seconds) and elongation (MIC: 90 seconds, 72°C, IL-15 and IL-15Rα: 60 seconds 72°C)

As controls, there were used primers specific to G3PDH: sense: 5'-GCCACCCAGAAGACTGTGGATGGC-3' (SEQ ID No: 8) and anti-sense: 5'-CATGTAGGCCATGAGGTCCACCAC-3' (SEQ ID No: 9).

(D) Statistical analysis

Data were represented by average + SD, and compared by Bonferroni test using ANOVA.

When p value was <0.01, a difference was believed to be significant.

Example 1-1 Induction of MICA/B expression by cytokine

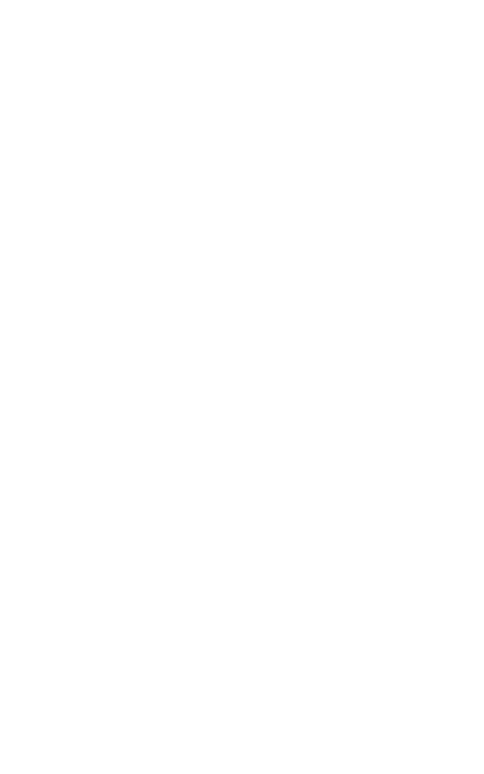

Whether MICA/B is induced by various cytokines or not was investigated in DCs derived from healthy donors and HCV-infected patients having or not having ALT abnormality. Monocyte-derived DCs from healthy donors (N-DC) and HCV-infected patients (HCV-DC) were prepared, and at day 6 of culturing, stimulated for 24 hours with IFNα (1000 U/mL), IFNβ (1000 U/mL), TNFα (10 ng/mL), IL-12 (10 ng/mL), IL-15 (50 ng/mL) and IL-18 (20 ng/mL). Then, MICA/B expression was analyzed by flow cytometry. The results are shown in Fig. 1.

DCs from normal donors (N-DC) responded to IFNα or IFNβ to express MICA/B, however, DCs from HCV-infected patients (HCV-DC) did not express MICA/B in any cases. IL-15 could apparently induce MICA/B, in N-DC and HCV-DC. In contrast, TNFα, IL-12 or IL-18 did not induce MICA/B expression.

Example 1-2 RT-PCR analysis of MICA/B mRNA

Total RNAs were isolated from DCs not stimulated, DCs stimulated with IL-15 (50 ng/mL) and DCs stimulated with IFNα (1000 U/mL), respectively, and expression of MICA mRNA and expression of MICB mRNA were analyzed by RT-PCR. Consequently, results corresponding to the above-mentioned results of flow cytometry were obtained.

Example 1-3 Difference of MICAB expression depending on presence or absence of ALT abnormality

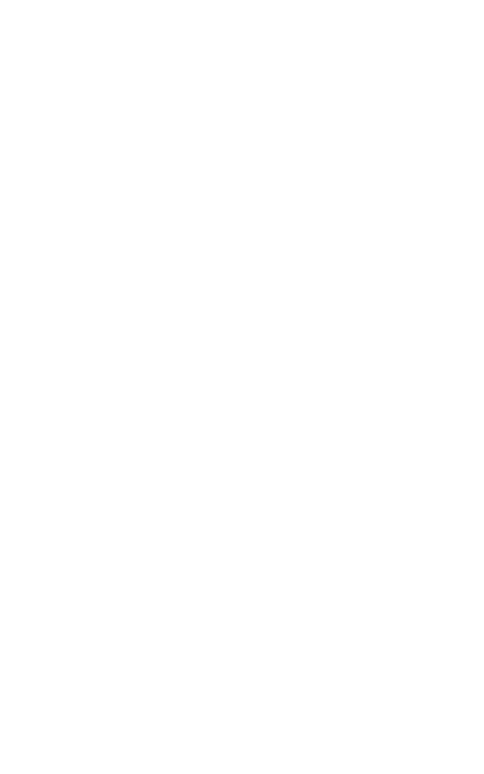

MICAB expression was compared among healthy donors (n=15)(HV), chronic HCV-infected patients showing abnormal ALT (n=10)(CH-1) and chronic HCV-infected patients having persistent normal ALT level (n=10)(CH-2). MICAB expression of DCs stimulated with IFNα or IL-15 was checked by flow cytometry, and represented as percent of positive cells. The results are shown in Fig. 2. In the drawing, a horizontal bar represents an average, a vertical bar represents SD, and * represents p<0.01. HCV-DC, when stimulated with IFNα, did not express MICA/B irrespective of the condition of ALT abnormality. This suggests that no MICA/B expression in response to IFNα or IFNβ in HCV-DC is not caused by chronic inflammation of liver.

Example 2 Evaluation of NK cell activation by DC stimulated with IL-15

Activation of NK cells by DC was evaluated using a cytolytic assay and an IFNγ expression assy.

NK cells were isolated from PBM of a healthy donor, and labeled with a concentrated antibody cocktail for NK cell, then, labeled with magnetic colloid (StemCell Technologies (Vancouver, BC, Canada)). 90% or more of the cells were CD56+CD3-lymphocytes. The concentrated NK cells were cultured with RPMI 1640 medium supplemented with 10% FCS and 10 U/mL penicillin/streptomycin, in a 24-well culture dish ($5.0 \times 10^5$/well). DCs from a healthy individual (N-DC) or a HCV-infected individual (HCV-DC) were stimulated with IFNα (1000 U/mL) or IL-15 (50 ng/mL) for 24 hours, added to a well at a concentration of $1.0 \times 10^5$/mL, and co-cultured together with NK cells for 24 hours.

The cytolysis assay was carried out as described below. Target cells (K562) labeled with $^{51}Cr$ were incubated for 4 hours at various effecter/target ratios, in NK/DC co-culturing (with or without trans well system) or NK cell single culturing. After incubation, the supernatant was recovered, and γ counting was conducted. Maximum release or natural release was defined as counting from a sample incubated with 5% Triton-X or a sample incubated with only the medium, respectively. The cytolysis activity was calculated according to the following formula:

$$\text{Lysis \%} = (\text{experimental value of release - natural release}) \times 100 / (\text{maximum release - natural release})$$

In all assays, natural release was less than 20% of maximum release.

Intracellular IFNγ expression of an NK cell was assayed as described below. NK cells cultured for 24 hours together with DC as described above were incubated together with 10 ng/ml PMA and 1 μmol/L ionomycin (Sigma Aldrich). Next, after incubation at 37°C for 4 hours in the presence of 1 μl/mL GolgiPlug (registered trademark)(BD-Phanmngen), NK cells were stained at 4°C for 30 minutes with a PE-labeled CD56 monoclonal antibody. The cells were treated with Cytofix/Cytoperm (registered trademark) buffering solution (BD-Pharmingen) at room temperature for 15 minutes, then, stained with a FITC-labeled-anti-IFNγ monoclonal antibody (mouse IgG1). The stained cells were analyzed by flow cytometry. The number in right upper quadrant in flow cytometry indicates CD56 positive cells expressing IFNγ.

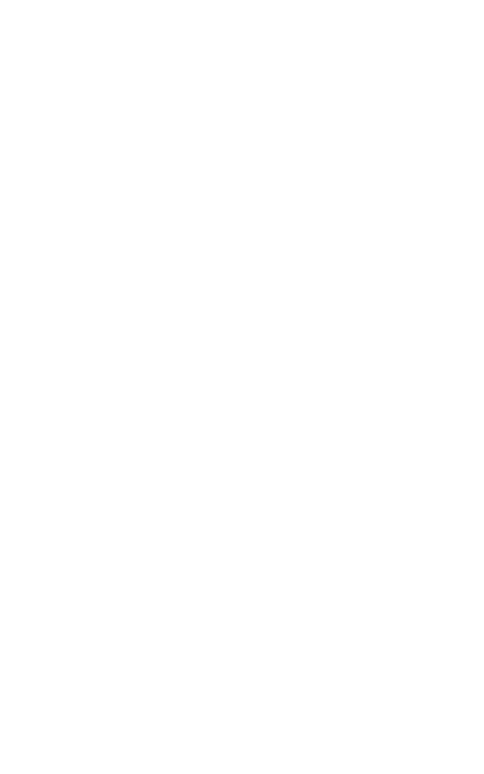

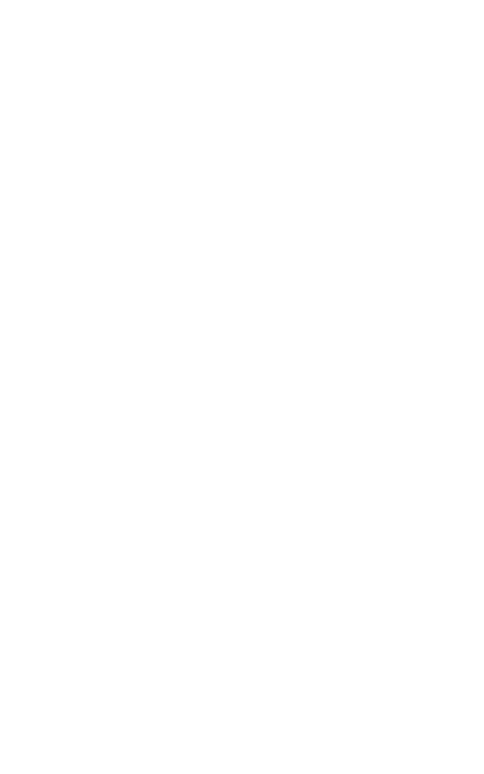

NK cells co-cultured with IL-15-stimulated N-DC and NK cells co-cultured with IFNα-stimulated DC showed increase in IFNγ production and in cytolytic activity for K562 cells at the analogous extent. In contrast, HCV-DC stimulated with IL-15 activated NK cells, while when stimulated with IFNα, did not activate NK cells (Figs. 3 and 4).

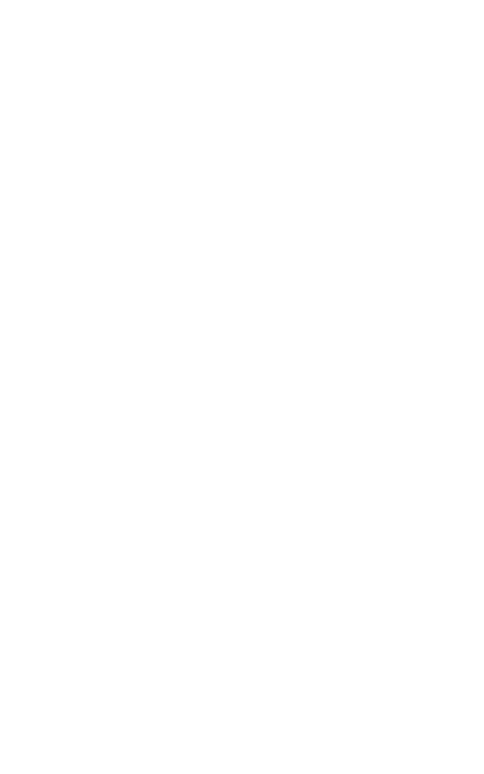

For comparison, newly isolated NK cells were cultured for 24 hours together with 50 ng/mL IL-15, then, K562 cytolytic activity and IFNγ production were assayed. As a result, IL-15 itself could not activate NK cells (Fig. 5). Therefore, it has been confirmed that IL-15 does not exert a direct effect on NK cells.

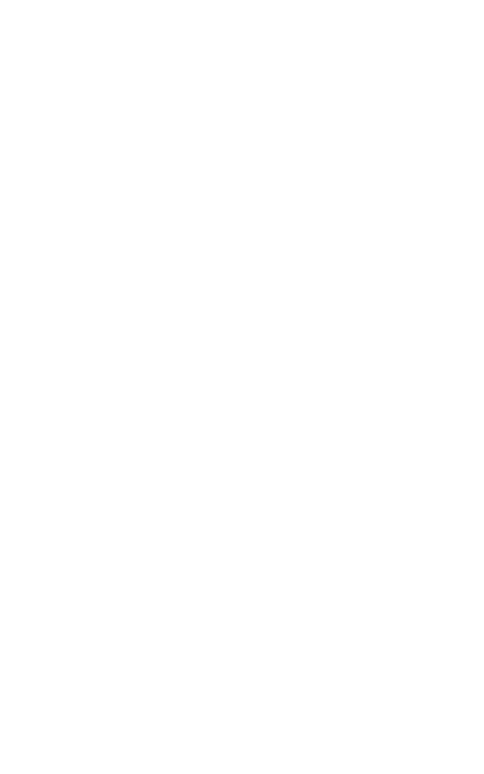

Next, newly isolated NK cells and N-DC stimulated with IL-15 (50 ng/mL) were separated using a 0.4 μm insertion membrane and co-cultured, and direct contact of NK cells and DC in the co-culture system could be prevented (trans well experiment). After culturing for 24 hours, K562 cytolytic activity was assayed. As a result, NK cells did not show increase in cylolytic ability. This shows that cell-cell direct contact is inevitable for activation of NK cells by N-DC stimulated with IL-15 (Fig. 6).

Example 3 Correlation of MICA/B-NKG2D mutual action in NK cell activation

Whether MICA/B expressed by DC stimulated with IL-15 is correlated with NK cell activation or not was checked.

NK cells were co-cultured for 24 hours together with N-DC or N-DC stimulated with IL-15, in the presence of an anti-MICAB monoclonal antibody (6D4), anti-NKG2D monoclonal antibody (1D11)(Science 285: 727, 1999; Proc. Natl. Acad. Sci. USA, 96: 6879, 1999) or control IgG, and cytolytic activity of NK cells and IFNγ production were checked.

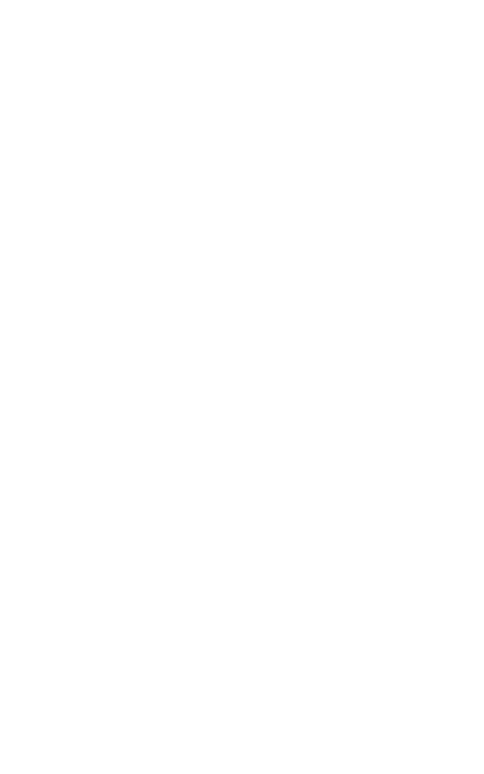

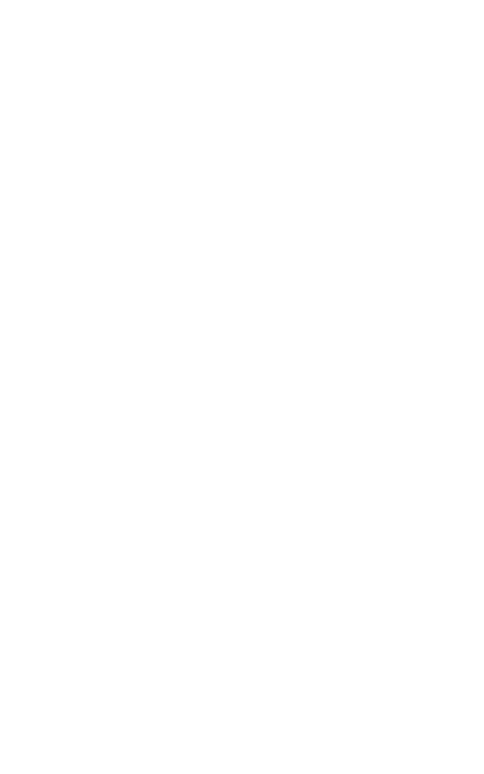

NK cells co-cultured with IL-15-stimulated N-DC, in the presence of an anti-MICA/B monoclonal antibody did not show increase in cytolytic ability (Fig. 7). Increase of IFNγ production of NK cells by DC stimulated with IL-15 disappeared completely when an anti-MICA/B monoclonal antibody or an anti-NKG2D monoclonal antibody was added during NK/DC co-culturing (Fig. 8). The same result was obtained when HCV-DC was used instead of N-DC (data not shown). These results indicate that activation of NK cells by DC stimulated with IL-15 depends on MICA/B-NKG2D mutual action.

Example 4 Defect of production of IL-15 in response to IFNα/β in HCV-DC

N-DC and HCV-DC were stimulated with IFNα (1000 U/mL) for 24 hours(n=5 for each group). IL-15, IL-12p70, TNFα and IL-1β in each culture supernatant were measured by solid phase sandwich ELISA using a couple of specific monoclonal antibodies and a recombinant cytokine standard substance (IFNα/β, IL-12p70, TNFα and IL-1β, Endogen, Wobum, MA; IL-15, BD-Pharmingen). The limit thresholds of detection of these ELISA systems are as described below: IL-15, 3.7 pg/mL; IFNα/β, 17.5 pg/mL; IL-12p70, TNFα and IL-1β, 8.8 pg/mL.

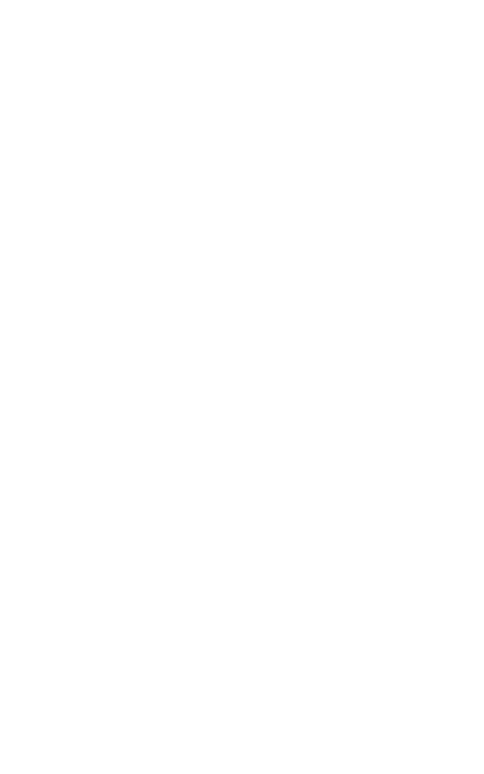

IFNα induced apparently production of IL-15 in N-DC, however, could not induce production of IL-15, in HCV-DC. Productions of IL-12, TNFα, IL-6 and LI-1β showed almost no difference between N-DC and HCV-DC (Fig. 9). The same result was obtained also when expression of IL-15mRNA was measured by RT-PCR (data not shown).

These results show that production of IL-15 is induced by stimulation with IFNα in N-DC, while not induced in HCV-DC.

Example 5 Engagement of autocrine IL-15 in MICA/B expression of DC stimulated with IFNα

IL-15 is known to manifest its biological action by binding to a trimer IL-15 receptor complex constituted of a specific α-chain for IL-15 (IL-15Rα), IL-2 receptor β-chain and common γ-chain. For investigating whether IFNα regulates expression of IL-15Rα in human DC or not, expression of mRNA of IL-15Rα was checked by semi-quantitative RT-PCR. Though IL-15Rα mRNA is expressed even in DC not stimulated with IFNα, when stimulated with IFNα, IL-15Rα mRNA was up-regulated in both N-DC and HCV-DC (data not shown). This shows that expression of IL-15 responding to IFNα has a defect but expression of IL-15Rα has no defect, in HCV-DC.

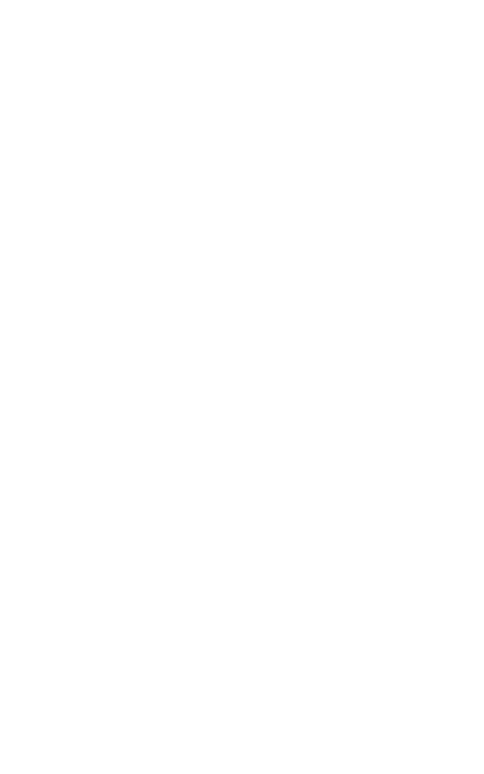

N-DC was cultured for 24 hours together with 1000 U/ml IFNα in the presence or absence of an anti-IL-15 antibody (30 μg/mL) or an anti-IL-15Rα neutralization antibody (30 μg/mL), then, MICAB expression was analyzed by flow cytometry. The results are shown in Fig. 10. In the drawing, the number in each histogram represents an average fluorescent intensity of MICA/B expression.

Expression of MICA/B in N-DC stimulated with IFNα was inhibited completely by any antibodies.

These results show that activation of IL-15Rα mediated by autocrine IL-15 is essential for MICA/B expression induced by IFNα, in DC. Namely, HCV-DC cannot express MICA/B in response to IFNα, and accordingly, it is believed that no activation ofNK cells is caused by deficiency of IL-15 production.

Example 6 Engagement of autocrine IFNα/β in IL-15-mediating MICA/B expression of DC

Whether autocrine IFNα/β is correlated with induction of MICA/B in DC stimulated with IL-15 or not was investigated.

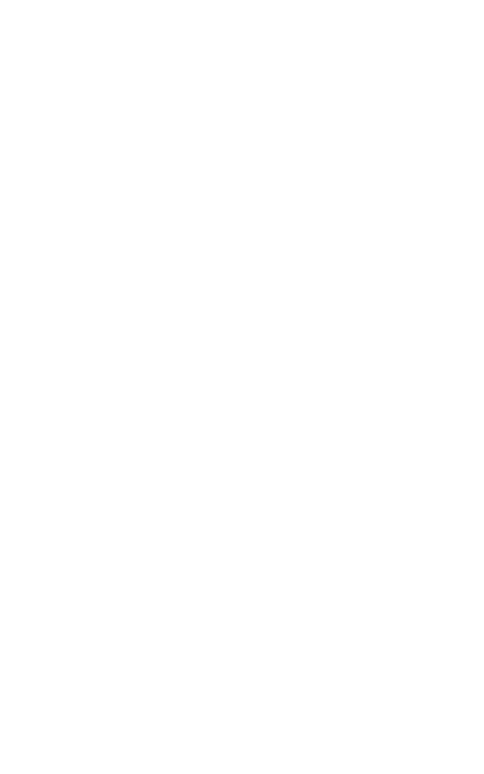

N-DC and HCV-DC were cultured for 24 hours together with 50 ng/ml IL-15 in the presence or absence of an anti-IFNα/βR neutralization antibody (30 μg/mL)(n=5 for each group). IFNα and IFNβ in each culture supernatant were measured by ELISA. The results are shown in Fig. 11.

In any of N-DC and HCV-DC, both IFNα and IFNβ were produced when stimulated with IL-15. Further, when DC is stimulated with IL-15 in the presence of an anti-IFNα/βR antibody, production of IFNα is substantially suppressed, while production of IFNβ is not suppressed (Fig. 11). This suggests that when DC is stimulated with IL-15, IFNβ is produced, then, IFNα is produced in a IFNα/βR-dependent manner.

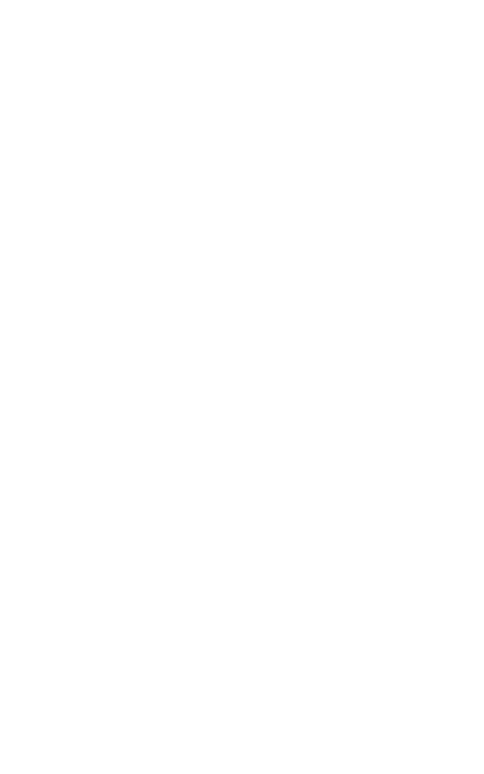

Next, N-DC and HCV-DC were stimulated for 24 hours with 50 ng/ml IL-15 in the presence or absence of an anti-IFNα/βR neutralization antibody (30 μg/mL), then, MICA/B expression was analyzed by flow cytometry. The results are shown in Fig. 12. In the drawing, the number in each histogram represents an average fluorescent intensity of MICA/B expression. IL-15-inducing MICA/B expression in DC was suppressed completely in the presence of an anti-IFNα/βR masking antibody. The same result was shown also in analysis of MICAB mRNA expression by RT-PCR (data not shown).

This shows that autocrine IFNα/β is necessary for expression of MICA/B responding to IL-15 in DC, and this is preserved also in HCV-infected patients. This result coincides with the result of Example 1 in which also HCV-DC can express MICA/B when stimulated with IL-15. That is, it is believed that IL-15 activates DC to produce IFNα/β, and this has an autocrine action necessary for MICA/B expression.

In Example 7-12, the following reagents were used: Recombinant human IL-4 and GM-CSF were obtained from PeproTech (London, UK), recombinant human soluble CD40 ligand (CD40L), human TNF-α and IL-3 from R&D Systems (Minneapolis, MN), LPS, polyI:C, mannan and galactose from Sigma (St. Louis, MO), recombinant human IFN-γ from Strathman Biotech GmbH (Hamburg, Germany), human lymphoblast-like IFN-α from Sumitomo Pharmaceuticals (Osaka, Japan), non-methylated CpG oligo deoxynucleotide (ODN) 2006 from sigma Genosys (Hokkaido, Japan), respectively. The following FITC-, PE-, PerCP- or PC5-conjugated anti-human monoclonal antibody were used: Lineage (Lin)(CD3, CD14, CD16, CD19, CD20, CD56)(Becton Dickinson), CD1a (NA1/34; DAKO, Glostrup, Denmark), CD11c (KB90; DAKO), CD14 (M5E2; Becton Dickinson), CD40 (5C3; BD Pharmingen, San Diego, CA), CD80 (L307.4; BD Pharmingen), CD83 (HB15a; Immunotech, Marseille, France), CD86 (IT2.2; B70/B7-2, BD Pharmingen), CDw123 (7G3; IL-3 receptor α-chain, BD Pharmingen), CD206 (mannose receptor, 3.29B1, 10; Imunotech), CD207 (langerin, DCGM4; Imunotech), DC-SIGN (120507; R&D Systems) and HLA-DR (L243; Becton Dickinson).

Example 7 Measurement of the number of MDC, PDC and DC precursor cell in peripheral blood of HCV-infected patient

The number of DC of 66 HCV-infected patients was measured. It was confirmed that these patients were positive for both serum anti-HCV antibody and HCV-RNA and negative for other virus infections, for example, hepatitis B virus (HBV) and HIV. No patient has been treated with an anti-virus agent, for example, IFN-$\alpha$ or ribavirin. Patients having other causes of hepatic diseases, for example, autoimmune, alcoholic disease or metabolic liver disease were excluded. In all patients, no hepatic cirrhosis and no hepatic carcinoma were confirmed using biochemical and ultrasonic wave examinations or CAT scan analysis in combination. In contrast, age-matched 19 healthy donors negative to all of HCV, HBV and HIV were also investigated.

Disease conditions of patients were evaluated every 1 to 2 months. Based on a pattern of abnormality of a liver function test, these patients were classified into two groups. Patients showing persistent normal alanine amino transferase (ALT) level for 2 years or more were defined to be included in an asymptomatic carrier (ASC) group. The remaining patients showed persistent or varying ALT abnormality and were defined to be included in a chronic hepatitis (CH) group. In some of these patients, liver biopsy was performed, and histological activity and grade of fibrosis were diagnosed. In ASC patients, only minimum monocyte infiltration was observed at a portal region, and apparent lobe inflammation or necrosis was not observed. These fmdings constituted a clear contrast with CH patients, and CH patients showed inflammation of higher extent which ranges from a portal region to vein regions and has lobe necrosis in the form of dot. The amount of HCV-RNA was assayed by branched DNA probe assay (Chron HCV-RNA, Emeryville, CA). HCV serotype typing was conducted by a known method. Patient profiles are shown in Table 1.

Table 1 Clinical background* of healthy donor and HCV-infected patient

| | Healthy donor (NV) | Asymptomatic carrier (ASC) | Chronic hepatitis (CH) |
|---|---|---|---|
| N | 23 | 14 | 43 |
| Sex (M/F) | 20/3 | 6/8 | 27/16 |
| Age | 45±10 | 46±10 | 48±8 |
| ALT | - | 21±5 | - |
| HCV-RNA titer | - | 3.5(0-35) | 5.7 (0-37) |
| HCV serotype (1/2/unclassified) | - | 5/ | 27/2/ |
| * value is represented by average ± SD ALT, alanine amino transferase<br>** p<0.01 vs. ASC<br>*** median (range) is represented by million genome equivalent/ml (Meq/ml) | | | |

10 to 12 ml of heparin-added venous blood was obtained from patients and healthy donors. Peripheral blood monocytes (PBMC) were recovered by density gradient centrifugation on Ficoll-Hypaque cushion. Live PBMCs were counted, then, the cell was dyed with an antibody. Blood DC was defined as a cell negative for lineage marker (Lin) (CD3, CD14, CD16, CD20, and CD56) and positive for HLA-$DR^+$. A gate of these cells was set, then, MDC and PDC were further defined depending on a pattern of expression of CD11c and CD123. MDC is a cell of $Lin^-$, HLA-$DR^+$, $CD11c^+$ and $CD123^{low}$, and PDC is a cell of $Lin^-$, HLA-$DR^+$, $CD11c^-$ and $CD123^{high}$. MDC and PDC are derived from a common hematopoietic stem cell, and subsequently differentiated into myeloid and lymphocyte-like DC precursor cells, respectively, and a phenotype of intermediate PDC precursor cells between the processes is determined. A CD34 precursor cell was defined as a cell of $Lin^-$, HLA-$DR^+$, $CD123^+$ and $CD34^+$. An early stage precursor cell and a late stage precursor cell of PDC were defmed as a cell of $Lin^-$, $CD34^+$, $CD123^+$ and $CD45RA^-$ and a cell of $Lin^-$, $CD34^+$, $CD123^+$ and $CD45RA^+$, respectively. The absolute number of DC subset in peripheral blood was calculated by multiplying the percentage of these cells determined by FACS analysis by the number of PBMC.

For functional analysis, some degree of alteration was applied (19) by BDCA-1 and BDCA-4 separation kits (MiltenyiBiotec, Bergisch, Germany), and MDC and PDC were separated from PBMC or buffy coat (Osaka Red Cross Blood Center). Alternatively, $Lin^+$ cells were magnetically depleted by a cocktail antibody comprising CD3, CD14, CD16, CD20, CD56 and glycophorin (Stem Cell Technologies, Vancouver, Canada), then, MDC and PDC were separately sorted by FACS Vantage SE (Becton Dickinson Immunocytometry Systems, San Jose, CA).

The results are shown in Fig. 13. In the graph, a horizontal line represents median. The total number of DC and PDC in the CH group was lower than the total number in the normal volunteer (NV), but was not different from the total number in the ASC group. MDC in the CH group tended to be fewer than in ASC and NV. For excluding a possibility that reduction of DC subset is ascribable to reduction of total PBMC in a HCV-infected patient, the numbers of PBMC, CD4 and monocyte were compared between the groups, to find no difference (data not shown). Namely, a small number of blood DC is not ascribable simply to a small number of PBMC but ascribable to selective reduction of DC in a CH patient.

Next, the number of precursor cells of DC subset was analyzed. CD34 precursor cells ($Lin^-$, $HLA-DR^+$, $CD123^+$, $CD34^+$), early stage precursor cells ($Lin^-$, $CD123^+$, $CD34^+$, $CD45RA^-$) and late stage precursor cells ($Lin^-$, $CD123^+$, $CD34^+$, $CD45RA^+$) were discriminated in PBMC according to phenotypes reported by Blom et al. (J. Exp. Med. 192: 1785-1796, 2000). The numbers of the CD34 precursor cells and the early stage precursor cells in the CH group were significantly lower than those in the donor group, but those in the ASC group were not different from those in the donor group. These results show that blood DC and its precursor cell decrease in a chronic hepatitis C patient.

Example 8 Separation of DC cell from peripheral blood monocyte (PBMC) and culturing

A buffy coat was isolated from venous blood of a healthy volunteer, and PBMC was recovered from the buffy coat by Ficoll-Hypaque density gradient centrifugation. Monocyte, B cell, MDC and PDC were magnetically isolated using CD14-micro beads, CD19-micro beads, BDCA-1 and BDCA-4 DC isolation kits (Miltenyi Biotec, Bergish-Gladbach, Germany), respectively. CD4, CD8 T cell and NK cell were separated using respectively corresponding Stem-Sep kits (Stem Cell Technologies Inc, Vancouver, BC) from PBMC. CD34+ hematopoietic precursor cells were isolated from umbilical cord blood monocytes using CD34-micro beads (Miltenyi).

Expression of surface molecules on DC was analyzed by flow cytometry using FACS Caliber (Becton Dickinson, san Jose, CA). For staining, DC was kept at 4°C for 30 minutes together with a specific antibody or isotype antibody in PBS comprising 2% BSA and 0.1% sodium azide. MCD is a cell of $Lin^-$, $HLA-DR^+$, $CD11c^+$ and $CD1231^{low}$, and PDC is a cell of $Lin^-$, $HLA-DR^+$, $CD11c^+$ and $CD123^{high}$, and expression of CD40, CD80 and CD83 in these cells was low. The purities of all the isolated cells were higher than 90%.

The isolated monocyte or MDC was supplemented with 10% FCS, 50 IU/ml penicillin, 50 μg/ml streptomycin, 2 mM L-glutamine, 10 mM Hepes buffering solution and 10 μM nonessential amino acid (complete medium, CM), and cultured for 4 to 7 days in IMDM (GIBCO Laboratories, Grand Island, NY) comprising 50 ng/ml GM-CSF and comprising or not comprising 10 ng/ml IL-4. PDC was cultured for 4 days in the presence of 50 ng/ml IL-3 in CM.

Example 9. Evaluation of invasion of pseudo type VSV into cell

For clarifying which blood cell is sensitive for HCV infection, pseudo type VSV-comprising chimera HCVE1 and E2 proteins were used. Pseudo type VSV is constituted of recombinant VSV in which a glycoprotein (G) gene is substituted by a reporter gene coding GFP, and has chimera HCV E1 and E2 proteins as an envelope (VSV-E1E2)(J. Exp. Med.; 197(1): 121-127, 2003).

The virus was purified by centrifugal separation at 4°C for 2 hours at 25,000 rpm at 20%(v/w)-60%(v/w) non-continuous sucrose gradient by SW28 rotor (Beckman Coulter Inc., Fullerton, CA), and preserved at -80°C.

For determining the copy number of RNA in a virus sample, TaqMan EZ RT-PCR kit (PE Applied Biosystems, FosterCity, CA) was used. A forward primer (5'-cattattatcattaaaaggctc-3' (SEQ ID No: 10)) and a reverse primer (5'-gatacaaggtcaaatattccg-3' (SEQ ID No: 11)) which amplify a 323-bp segment of pseudo type VSV RNA, and a dual fluorescent group labeling probe [5'-(6-carboxy-fluorescein)-atccagtggaatacccggcagattac-(6-carboxy-tetramethyl-rhodam ine)-3' (SEQ ID No: 12)] were used. Release of fluorescence during PCR amplification was monitored by a sequence detector (ABI Prism 7000, PE Applied Biosystems), and the copy number in a sample was determined based on a calibration curve made from in vitro synthesized pseudo type VSV RNA of known amount.

Pseudo type VSV was inoculated on various cells separated from PBMC or umbilical cord blood. As a negative control, VSVΔG having no envelope protein was used. As a positive control, VSVΔG-G supplemented with VSV-G protein was used. The separated various blood cells were used to give a preparation of $5\times10^4$ cells/well in CM in a 96-well culture plate. Next, this was inoculated with a pseudo type virus VSV-E1E2 ($1\times10^{12}$ RNA copies/well), VSVΔG ($1\times10^{12}$ RNA copies/well) or VSVΔG-G ($1\times10^{11}$ RNA copies/well), and incubated at 37°C for 16 hours. The infected cells ($GFP^+$ cell) were observed by a fluorescent microscope, and positive percentage was measured by FACS analysis. The net percentage of the infected cells is represented as described below.

Infection % = (% of $GFP^+$ cell when VSV-E1E2 or VSVΔG-G is used) - (% of $GFP^+$ cell when VSV-ΔG is used)

As a result, VSVΔG-G infected MDC, PDC, monocyte and CD34+ hematopoietic precursor cell, while VSV-E1E2 did not infect these cells. Namely, DC newly isolated from peripheral blood was not sensitive against pseudo type VSV-E1E2.

Example 10 Influence of differentiation or maturation of DC exerted on sensitivity against pseudo type VSV

For investigating an influence of differentiation or maturation of DC exerted on sensitivity against pseudo type VSV, MDC or monocyte was cultured together with IL-4 or without IL-4 in the presence of GM-CSF. At day 4, the phenotype was analyzed. As a result, MDC cultured together with GM-CSF and IL-4 showed high expression of CD1a, CD83 and CD86 as compared with that cultured together with only GM-CSF. This shows that IL-4 acted as a DC differentiation factor. In MoDC, a similar difference in phenotype was observed between the cell cultured together with GM-CSF and the cell cultured together with GM-CSF and IL-4 in combination. At day 4 of culturing in the presence of IL-3, PDC showed higher expressions of CD40, CD80, CD83 and CD86 as compared with those at day 0.

These DCs were inoculated with pseudo type VSV-E1E2 and the results are shown in Fig. 14 and Fig. 15. In the drawings, a number in right lower quadrant represents a percentage of $GFP^+$ cells. In MDC at day 4 and in MoDC at days 4 and 7, $GFP^+$ cells were observed, while were not observed in PDC at day 4. In the case of inoculation of VSVΔG-G, positive signals were obtained from MDC at day 4, PDC at day 4 and MoDC at days 4 and 7 irrespective of difference of the cytokines used. In MDC at day 4 cultured together only with GM-CSF, the proportion of $GFP^+$ cells (n=3, median and range) was 1.5% (0.5 to 2.27%), while in that cultured together with GM-CSF and IL-4, $GFP^+$ cells of higher percentage were observed (23.3%, (12.3 to 27.0%)). MoDC showed various sensitivities against VSVΔG-G and VSV-E1E2 according to the persistent time of culturing. MoDC at day 4 showed VSV-E1E2-containing $GFP^+$ cells of higher percentage as compared with Mo-DC at day 7. The results described above showed that immature MDC and monocyte-derived DC are sensitive against pseudo type VSV

For verifying reliability of a pseudo type VSV system in evaluation of E1E2-mediating virus invasion, HCV-RNA in each DC after inoculation of serum of window period from a hepatitis C patient was quantified by real time PCR. In the experiment, a commercially available HCV sero-conversion panel was used as an inoculation substance. This has high HCV RNA titer ($1\times10^5$ copies/μl), and does not contain an anti-HCV antibody (Bio Clinical Partners, Inc, USA). 3 μl/well inoculation substance was added to DC in a 96-well plate, and incubated at 37°C for 24 hours. DC was recovered and washed thee times with IMDM supplemented with 1% FCS, then, the total RNA was extracted from DC using RNeasy Mini Kit (QIAGEN, Germany).

For measuring HCV RNA, TaqMan EZ RT-PCR kit (PEApplied Biosystems) was used. A forward primer (5'-cgggagagccatagtgg-3' (SEQ ID No:. 13)) and a reverse primer (5'-cgaaaggccttgtggtact-3' (SEQ ID No:. 14)) which amplify a 161-bp segment in 5'-non-coding region of HCV RNA, and a dual fluorescent group labeling probe [5'-(6-carboxy-fluorescein)-ctgcggaaccggtgagtacac-(6-carboxy-tetramethyl-rhodamine)-3' (SEQ ID No:. 15)] were used. Release of fluorescence during PCR amplification was monitored using a sequence detector (ABI Prism 7000), and the copy number in a sample was determined based on a calibration curve made from in vitro synthesized HCV RNA of known amount.

Among the cells checked, highest HCV RNA titer was detected in MDC at day 4 cultured together only with GM-CSF (Fig. 16). This coincides with the result obtained by using pseudo type VSV. Therefore, it was confirmed that the data using a pseudo type VSV system correctly reflects sensitivity of a cell against true HCV

Example 11 Influence of MDC maturation stimulator exerted on pseudo type VSV infection

To find a substance for preventing DC from HCV infection, MDC was treated with various maturation factors in an inoculation experiment. To find a substance having a possibility of preventing DC from HCV infection, invasion of VSV-E1E2 into a cell was checked for cells treated with IFNα, IFNγ, CpG ODN 2006, CD40L, polyI:C, TNFα or LPS and cells not treated. DC was separated, and IFNα (100 U/ml), IFNγ (1000 U/ml), IL-3 (50 ng/ml) and IL-4 (10 ng/ml) were added to DC, respectively, on the same day. CpG ODN 2006 (10 μM), CD40L (1 μg/ml), polyI:C (50 μg/ml), TNFα (20 μg/ml) and LPS (10 μg/ml) were added to DC 24 hours before inoculation of a pseudo virus.

The results are shown in Fig. 17. It has been clarified that when IL-4 and CpG ODN 2006 are added to a medium, sensitivity against VSVΔG-G is not influenced and the percentage of VSV-E1E2-containing $GFP^+$ cells decreases significantly, as compared with MDC cultured in GM-CSF. On the other hand, IFN-α, polyI:C, LPS and TNF-α decreased the percentage of $GFP^+$ cells for both VSV-E1E2 and VSVΔG-G. It has been clarified by phenotype analysis that CpG ODN up-regulated expression of CD83 and CD86 in MDC cultured together with GM-CSF. These results show that sensitivity against pseudo type VSV-E1E2 was lost by stimulating maturation of MDC.

Example 12 Influence of lectin-binding substance exerted on pseudo type VSV infection

For investigating whether a lectin-containing molecule on DC is correlated with HCV infection or not, mannan and galactose were tested for disturbance of invasion of VSV-E1E2. MDC cultured together with GM-CSF was incubated at 37°C for 180 minutes together with mannan and galactose of various concentrations at day 4, and pseudo type VSV was inoculated. DC was also treated with EDTA(5mM) before pseudo type VSV inoculation. For comparing DC and a hepatoma cell strain HepG2, HepG2 was treated with 5 μg/ml mannan before pseudo type VSV inoculation.

When MDC was pre-treated with mannan, the percentage of $GFP^+$ cell having VSV-E1E2 decreased in dose-dependent manner, while invasion of VSVΔG-G was not influenced (Fig. 18). Such disturbing effect of mannan was confirmed in MDC inoculated with true HCV (Fig. 19). In contrast, galactose did not exert an influence on infection in MDC for both VSV-E1E2 and VSVΔG-G. Interestingly, EDTA did not decrease the sensitivity of VSV-E1E2, but completely excluded the sensitivity of VSVΔG-G. These data show that a mannose type carbohydrate is correlated with a mutual action between DC and VSV-E1E2 in $Ca^{2+}$ non-dependent manner. In contrast, in HepG2 cells, mannan did not inhibit infection of VSV-E1E2 (Fig. 20). This shows that molecules contributing to invasion of VSV-E1E2 are different in HepG2 and MDC, further that mannan exerts an influence on a molecule on MDC, but no influence on a molecule on VSV-E1E2.

For investigating whether expression of lectin in DC is parallel to its sensitivity against VSV-E1E2, expressions of DC-SIGN, mannose receptor (MR) and langerin were compared, for MDC cultured together with GM-CSF and MDC cultured together with GM-CSF and IL-4 in combination. Expression of DC-SIGN was higher in MDC cultured together with GM-CSF and IL-4 than in MDC cultured together with GM-CSF, but expressions of MR and langerin were not different. These results show that expression of lectin on DC is not directly correlated with infection of VSV-E1E2.

## INDUSTRIAL APPLICABILITY

The present invention provides a pharmaceutical composition for treating chronic hepatitis C, comprising novel active components effective for treatment of chronic hepatitis C (IL-15, myeloid dendritic cell maturation stimulator and/or lectin-binding substance), and a method of treating chronic hepatitis C using these active components. By the pharmaceutical composition and treatment method of the present invention, persistently infected hepatitis C virus can be decreased, and by this, sideration of hepatic cirrhosis and hepatic carcinoma can be prevented in a chronic HCV-infected patient.

## Claims

1. A pharmaceutical composition for treating chronic hepatitis C, comprising at least one active ingredient selected from the group consisting of IL-15, myeloid dendritic cell maturation stimulators and lectin-binding substances.

2. The pharmaceutical composition according to Claim 1, comprising IL-15.

3. The pharmaceutical composition according to Claim 2 for use together with IFN-α in treatment of chronic hepatitis C, comprising IL-15.

4. The pharmaceutical composition according to Claim 1, comprising a myeloid dendritic cell maturation stimulator.

5. The pharmaceutical composition according to Claim 4 for use together with IFN-α in treatment of chronic hepatitis C, comprising a myeloid dendritic cell maturation stimulator.

6. The pharmaceutical composition according to Claim 4 or 5, wherein the myeloid dendritic cell maturation stimulator is selected from the group consisting of CpG oligo deoxynucleotide, GM-CSF, IL-4, LPS, CD40L, polyI:C, TNF-α and IFN-γ.

7. The pharmaceutical composition according to Claim 1, comprising a lectin-binding substance.

8. The pharmaceutical composition according to Claim 7 for use together with IFN-α in treatment of chronic hepatitis C, comprising a lectin-binding substance.

9. The pharmaceutical composition according to Claim 7 or 8, wherein the lectin-binding substance is selected from the group consisting of mannose carbohydrates, fucose carbohydrates and anti-lectin antibodies.

10. A pharmaceutical composition for preventing hepatic cirrhosis or hepatic cell carcinoma, comprising at least one active ingredient selected from the group consisting of IL-15, myeloid dendritic cell maturation stimulators and lectin-binding substances.

**11.** A method of treating chronic hepatitis C, comprising administration to a patient of an effective amount of at least one active ingredient selected from the group consisting of IL-15, myeloid dendritic cell maturation stimulators and lectin-binding substances.

**12.** Use of IL-15, myeloid dendritic cell maturation stimulator and/or lectin-binding substance in manufacture of a medicine for treatment of chronic hepatitis C.

**INTERNATIONAL SEARCH REPORT**

International application No.
PCT/JP2004/013283

A. CLASSIFICATION OF SUBJECT MATTER
Int.Cl[7] A61K38/20, 38/21, 38/17, 31/7004, 31/7088, A61P1/16, 31/14

According to International Patent Classification (IPC) or to both national classification and IPC

B. FIELDS SEARCHED

Minimum documentation searched (classification system followed by classification symbols)
Int.Cl[7] A61K38/20, 38/21, 38/17, 31/7004, 31/7088, A61P1/16, 31/14

Documentation searched other than minimum documentation to the extent that such documents are included in the fields searched

| | | | |
|---|---|---|---|
| Jitsuyo Shinan Koho | 1922-1996 | Toroku Jitsuyo Shinan Koho | 1994-2004 |
| Kokai Jitsuyo Shinan Koho | 1971-2004 | Jitsuyo Shinan Toroku Koho | 1996-2004 |

Electronic data base consulted during the international search (name of data base and, where practicable, search terms used)
CAPLUS(STN), BIOSIS(STN), REGISTRY(STN), EMBASE(STN), MEDLINE(STN)

C. DOCUMENTS CONSIDERED TO BE RELEVANT

| Category* | Citation of document, with indication, where appropriate, of the relevant passages | Relevant to claim No. |
|---|---|---|
| A | JINUSHI, Masahisa *et al.*, Critical role of MHC class I-related chain A and B expression on INF-α-stimulated dendritic cells in NK cell activation: Impairment in chronic hepatitis C virus infection, Journal of Immunology, 2003 Febraury, Vol.170, pages 1249 to 1256, full text | 1-3,10,12 |
| A | FAWAZ, Lama M. *et al.*, Up-regulation of NK cytotoxic activity via IL-15 induction by viruses: A comparative study, Journal of Immunology, 1999, Vol.163, pages 4473 to 4480, full text | 1-3,10,12 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 October, 2004 (08.10.04) | 14 December, 2004 (14.12.04) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/013283

**Box No. II Observations where certain claims were found unsearchable (Continuation of item 2 of first sheet)**

This international search report has not been established in respect of certain claims under Article 17(2)(a) for the following reasons:

1. [X] Claims Nos.: 11
because they relate to subject matter not required to be searched by this Authority, namely:
Claim 11 pertains to methods for treatment of the human body by therapy.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
(See extra sheet.)

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [X] No required additional search fees were timely paid by the applicant. Consequently, this international search report is restricted to the invention first mentioned in the claims; it is covered by claims Nos.:
In the inventions according to claims 1, 10 and 12 of the present case, those wherein IL-15 is used as the active ingredient, and claims 2 and 3.

**Remark on Protest**

[ ] The additional search fees were accompanied by the applicant's protest.

[ ] No protest accompanied the payment of additional search fees.

Form PCT/ISA/210 (continuation of first sheet (2)) (January 2004)

**INTERNATIONAL SEARCH REPORT**

| International application No. |
|---|
| PCT/JP2004/013283 |

C (Continuation). DOCUMENTS CONSIDERED TO BE RELEVANT

| Category* | Citation of document, with indication, where appropriate, of the relevant passages | Relevant to claim No. |
|---|---|---|
| P,A | JINUSHI, Masahisa *et al.*, Autocrine/paracrine IL-15 that is required for type I IFN-mediated dendritic cell expression of MHC class I-related chain A and B is impaired in hepatitis C virus infection, Journal of Immunology, 2003 November, Vol.171, pages 5423 to 5429, full text | 1-3,10,12 |
| P,A | KANTO, Tetsuya *et al.*, IFN-gamma or IL-15 restores the impaired helper T-cell polarliz ing ability of blood dendritic cell subsets in chronic hepatitis C, Hepatology, 2003 October, Vol.38, No.4, Suppl.1, pp342A, full text | 1-3,10,12 |

Form PCT/ISA/210 (continuation of second sheet) (January 2004)

**INTERNATIONAL SEARCH REPORT**

International application No.
PCT/JP2004/013283

Continuation of Box No.III of continuation of first sheet(2)

When "a medicinal composition for treating chronic hepatitis C containing IL-15" is considered as a specified invention among the medicinal compositions containing three different active ingredients, which are described as alternatives, involved in "a medicinal composition for treating chronic hepatitis C containing at least one active ingredient selected from the group consisting of IL-15, myeloid dendritic cell maturation stimulators and lectin-binding substances" according to the invention as set forth in claim 1 of the present case, the common matter thereof to the medicinal compositions containing the two other active ingredients seemingly resides in "a medicinal composition for treating chronic hepatitis C".

However, a remedy for chronic hepatitis C comprising as the active ingredient INF-γ, which is a myeloid dendritic cell maturation stimulator, has been publicly known as reported by the following document. Thus, the above constitution cannot be recognized as a novel matter.

Moreover, these inventions have no technical problem in common that having been unsolved until the application of the present case.

Such being the case, the inventions using a myeloid dendritic cell maturation stimulator or a lectin-binding substance as the active ingredient, among the inventions according to claims 1 to 10 and 12 in the present case, cannot be considered as a group of inventions so linked as to form a single general inventive concept together with the specified invention as described above.

Document: WO 99/18993 A1 (Otsuka Pharmaceutical Co., Ltd.),
22 April, 1999 (22.04.99)

Form PCT/ISA/210 (extra sheet) (January 2004)

SEQUENCE LISTING

<110> Intellectual Property Consulting Incorporated

<120> Composition for Treatment of Chronic Hepatitis C

<130> P04-145PCT

<150> JP 2003-315498
<151> 2003-09-08

<160> 15

<170> PatentIn version 3.1

<210> 1
<211> 20
<212> DNA
<213> homo sapiens

<400> 1
acacccagca gtggggggat 20

<210> 2
<211> 21
<212> DNA
<213> homo sapiens

<400> 2
gcagggaatt gaatcccagc t 21

<210> 3
<211> 21
<212> DNA
<213> homo sapiens

<400> 3
agcagtcgtg agtttgccca c 21

<210> 4
<211> 18
<212> DNA
<213> homo sapiens

<400> 4
taaaacagaa gccaactg 18

<210> 5
<211> 20
<212> DNA
<213> homo sapiens

<400> 5
caagaagtgt tgatgaacat 20

<210> 6
<211> 21
<212> DNA

<213> homo sapiens

<400> 6
gtcaagagct acagcttgta c 21

<210> 7
<211> 19
<212> DNA
<213> homo sapiens

<400> 7
ggtgagcttt ctcctggag 19

<210> 8
<211> 24
<212> DNA
<213> homo sapiens

<400> 8
gccacccaga agactgtgga tggc 24

<210> 9
<211> 24
<212> DNA
<213> homo sapiens

<400> 9
catgtaggcc atgaggtcca ccac 24

<210> 10
<211> 22
<212> DNA
<213> vesicular stomatitis virus

<400> 10
cattattatc attaaaaggc tc 22

<210> 11
<211> 21
<212> DNA
<213> vesicular stomatitis virus

<400> 11
gatacaaggt caaatattcc g 21

<210> 12
<211> 26
<212> DNA
<213> vesicular stomatitis virus

<400> 12
atccagtgga atacccggca gattac 26

<210> 13
<211> 17
<212> DNA

```
<213>  hepatitis C virus

<400>  13
cgggagagcc atagtgg                                                    17


<210>  14
<211>  19
<212>  DNA
<213>  hepatitis C virus

<400>  14
cgaaaggcct tgtggtact                                                  19


<210>  15
<211>  21
<212>  DNA
<213>  hepatitis C virus

<400>  15
ctgcggaacc ggtgagtaca c                                               21
```